# EUROPEAN PATENT SPECIFICATION

(11) **EP 0 353 204 B1**
(45) Date of publication and mention of the grant of the patent: **02.02.1994**
(21) Application number: 89830324.3
(22) Date of filing: 12.07.1989
(51) Int. Cl.: A61B 17/11

(54) **Bowel-anastomosis-ring holder pincers**
Zange zum Festhalten eines Darmanastomoseringes
Pince pour obtenir und anneau d'anastomose d'intestin

(30) Priority: 18.07.1988 IT 901688
(43) Date of publication of application: 31.01.1990
(73) Proprietor: CYANAMID ITALIA S.p.A., I-95121 Catania (IT)
(72) Inventor: Donini, Ippolito Guiseppe, Prof., I-Ducentola di Voghiera (Ferrara) (IT)
(74) Representative: Taliercio, Antonio

(56) References cited:
- EP-A- 0 158 316
- US-A- 3 265 069
- US-A- 4 632 435

## Description

This invention broadly relates to surgical pincers and particularly concerns such pincers for manipulating and holding bowel anastomosis rings.

Among the technical procedures for bowel suturation, the pressure suturation can be mentioned, namely the pressure butt joining of the anastomosis ends carried out by means of annular devices of various shapes and sizes inserted into the bowel lumen. These devices usually consist of two half-shells and of a central core. The half-shells are introduced into the lumen of the bowel ends and their edges are subsequently clamped onto the central core by means of so-called tobacco-bag strings. Then the two half-shells are brought together and pressure closed, thus enabling the bowel walls to mutually contact and to cure. Thereafter, the ring assembly falls into the bowel lumen and is ejected or it shatters and is progressively eliminated, if the assembly is biodegradable.

The ring suture assemblies frequently mentioned as B.A.R. (Bowel Anastomosis Rings), as presently available, can be used solely in those anatomical areas where an easy surgical access and a particularly large operation room are available. In no way they can be directly positioned by the hands of the surgeon in deep, narrow and/or hardly accessible areas, such as the rectum (pelvis minor) and the esophagus.

Lack of a suitable instrument for use in these areas has been of the utmost importance in the prior art, because sealing of the anastomosis in these areas is a critical aspect for the very life of the patient.

EP-A-0 158 316 discloses devices, kits and methods for non-suture end-to-end and end-to-side anastomosis of tubular tissue members and specially developed instruments to handle anastomosis clips. The clips have a ring-shaped body part defining a substantially circular opening and opposed ends separable under spring pressure to enlarge said opening. The applicator as disclosed comprises a pair of opposite legs connected at one end and having support means remote from said one end to supportingly engage the opposed handling elements of said clips.

The drawbacks of said applicator are not only that it can be used only with the disclosed clips designed only for blood vassel anastomosis, but also that in no way it considers the problem of reaching deep, narrow or hardly accessible areas.

It is an object of this invention to cure this deficiency of the prior art and to make a mechanical instrument and particularly pincers available for insertion of bowel anastomosis rings, adapted to enable such ring assemblies to be easily inserted into hardly accessible organs, such as lower rectum or esophagus, as well as to eliminate any hand contamination.

This object is attained by means of pincers that, in their basic embodiment, have two pivotally connected arms and a terminal head portion, angled with respect to said arms and consisting of two jaws which form in combination with one another an elliptical shape, each of said jaws including a lower claw upon which an upper claw is fixed as a staple member, having the same elliptical curvature as the lower claw and slightly inwardly staggered with respect thereto.

In the pincers according to the invention, the upper claws have a circular cross-section and the lower claws have an internal surface with slightly inwardly concave profile.

From a geometrical view point, the circles inscribed into the two claw pairs and tangent to their internal profiles are concentric.

Further details and advantages of this invention will be apparent from the following description to be read with reference to the enclosed drawings wherein the preferred embodiments are illustratively but not restrictively shown and wherein:
Fig. 1 is a side elevation view of a pincers device according to this invention;
Fig. 2 is an enlarged side elevation view, showing the head portion of the pincers;
Fig. 3 is an enlarged plan view of the head portion of the pincers;
Fig. 4 is a fragmentary view, in partial cross-section along line A-A of Fig. 3, showing the cross-sectional profiles of the jaw claws of the pincers;
Fig. 5 is a view similar to the view of Fig. 1 showing a different embodiment with a more angled head portion.

Referring now to the drawings, it can be seen that the pincers of this invention have two pivotally connected arms 10, 11 and a terminal head portion which is the effective operational portion of the pincers.

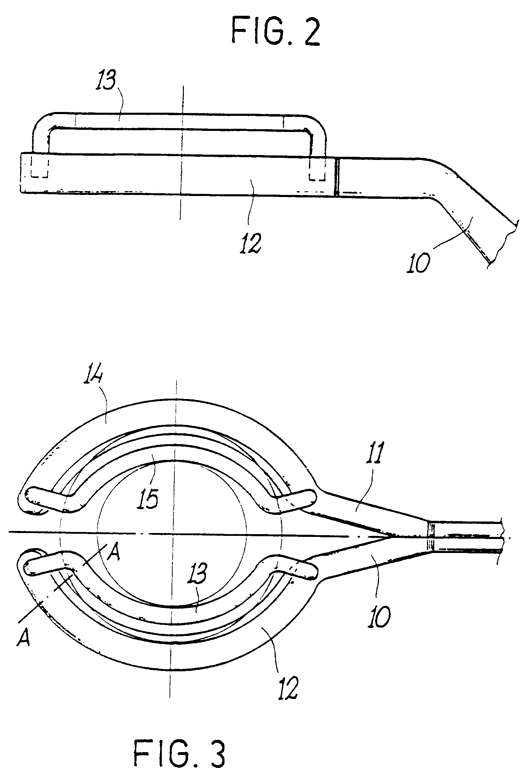

The operational portion, detailedly shown in Figs. 2 and 3, consists of two jaws which in combination form an elliptical shape, each including two superposed claws 12, 13 and 14, 15, respectively. The two upper claws 13 and 15 are fixed upon the two lower claws 12 and 14 as staple members and have the same elliptical curvature as the lower claws 12 and 14.

From a geometrical view point, it can be observed from Fig. 3 that, when jaws are in closed condition, the circles inscribed into the two lower claws 12, 14 and into the two upper claws 13, 15 and tangent to their internal profiles are concentric.

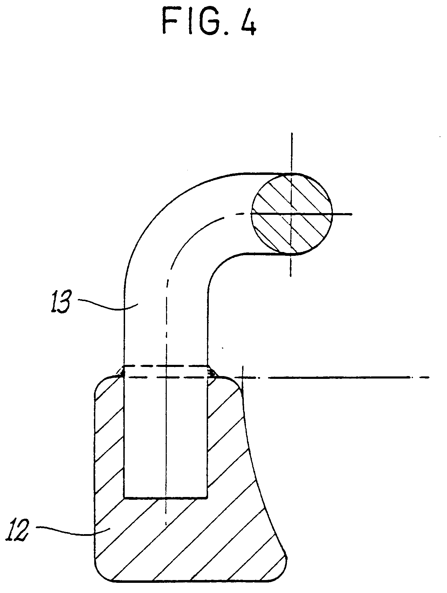

The cross-section of the upper claws 13, 15 is circular, while, as it can be seen in Fig. 4, the inner surface of the lower claws 12, 14 has a slightly inwardly concave profile such that, when the jaws are closed, the half-shell of the ring is grasped, pushed upwardly and anchored against the upper, slenderer claw, abutted to the central core of the ring. When this operation is carried out, after the first tobacco-ring string is closed upon the central core, sufficient room is left for the second tobacco-bag string to be closed. In addition, the pincers enable the half-shell to be substained in order to create the indispensable bearing point in absence of which it is not possible to pressure close the ring. This is useful particularly when anastomosis is to be carried out in deep areas (such as in rectum) or in restricted areas (such as in thoracic esophagus).

In conclusion, the pincers according to this invention noticeably enlarge the application range of the bowel anastomosis rings and in particular extend it so as to include all those areas where the application of such rings has a strategical relevance. In fact, when the anatomic-surgical conditions permit the rings to be manually positioned, then the employment of alternative as much safe techniques becomes even more possible.

Use of the pincers according to this invention in the rectum and in the esophagus is convenient under various technical aspects:
a- during the ring positioning step, the pincers enable the lower annular half-shell to be easily inserted into the rectum lumen (which is impossible to be manually carried out), in view of a grasping and fixing action upon the upper annular half-shell, already inserted into the colon lumen;
b - upon closure of the second tobacco-bag string, the ring is to be pressure tightened. This action is usually carried out by the surgeon who takes with his fingers the annular shells at four opposed points and exerts a noticeable pressure.

In the above mentioned areas, this action is effectively impossible due to shortage of room and to immobility of the organs, that do not enable the ring to be safely clasped with the fingers. This action becomes possible only when the pincers according to the invention are used as an "envil" to bear the distal (with respect to the hands of the surgeon) half-shell. The jaws of the pincers in closed condition act as a "seat" wherein the distal half-shell is positioned. In this manner, it is firmly supported, while the surgeon closes under pressure the proximal half-shell.

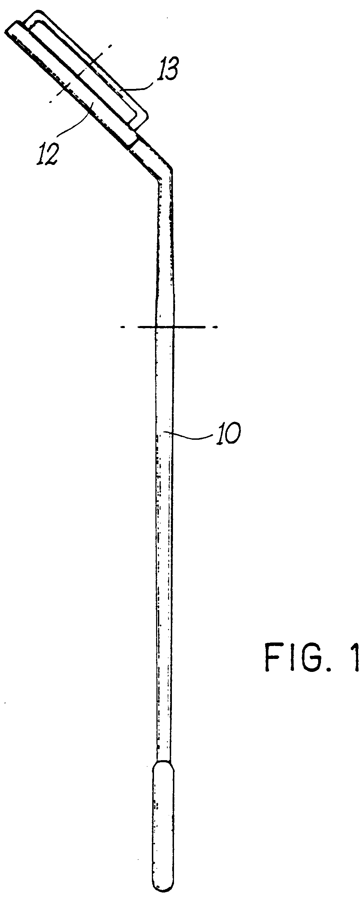

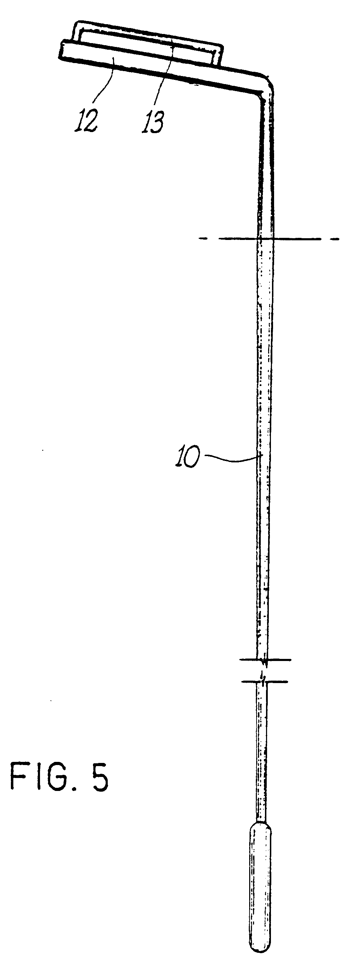

From the previous description, it will be easily understood that the angle of the head portion (jaws) with respect to the arms 10, 11 can be different from the one shown in Fig. 1, as it is shown in Fig. 5, and the arms can be longer or shorter to enable hardly accessible and deep areas to be reached. Furthermore, the pincers can be made of such a material as to be disposable.

## Claims

1. Bowel-Anastomosis-Ring holder pincers having two pivotally connected arms (10, 11) and a terminal head portion, angled with respect to said arms (10, 11), characterized in that said head portion consists of two jaws which form in combination with one another an elliptical shape, each of said jaws including a lower claw (12, 14) upon which an upper claw (13, 15) is fixed as a staple member, having the same elliptical curvature as the lower claw (12, 14) and slightly inwardly staggered with respect thereto and in that said upper claws (13, 15) have a circular cross section and said lower claws (12, 14) have an internal surface with a slightly inwardly concave profile.

2. Bowel-Anastomosis-Ring holder pincers according to claim 1, characterized in that, from a geometrical view point, the lower and upper claws (12,14; 13,15) have a curvature such that, in their closed condition, the circles inscribed therein and tangent to their internal profiles are concentric.

## Patentansprüche

1. Zange zum Festhalten eines Darmanastomoseringes, mit zwei gelenkig miteinander verbundenen Armen (10,11) und einem gegenueber diesen Armen (10,11) abgewinkelten Kopfendteil, dadurch gekennzeichnet, dass das vorgenannte Kopfendteil aus zwei untereinander eine elliptische Form bildenden Backen besteht, wobei jede Backe eine untere Kralle (12,14) enthaelt, an der eine obere Kralle (13,15) Brueckensteg befestigt ist, dass die gleiche elliptische Kruemmung als die untere Kralle (12,14) hat und gegenueber derselben leicht nach innen versetzt ist und dass die vorgennanten oberen Krallen (13,15) einen kreisfoermigen Querschnitt aufweisen und die vorgenannten unteren Krallen (12,14) eine innere, ein leicht nach innen ausgehoeltes Profil aufweisende Flaeche haben.

2. Zange zum Festhalten eines Darmanastomoseringes, dadurch gekennzeichnet, dass vom geometrischen Standpunkt aus, die untere und obere Krallen (12,14; 13,15) so gekruemmt sind, dass in deren geschlossenen Stellung, die in denen eingeschriebenen und zu deren Profile tangentialen Kreise zueinander konzentrisch sind.

## Revendications

1. Pince pour soutenir un anneau d'anastomose d'intestin ayant deux bras (10,11) articulés entre eux et une partie de tête terminale disposée en forme d'angle au regard desdits bras (10,11), caractérisée en ce que ladite partie de tête terminale consiste de deux mâchoires, qui forment en combination entre eux une forme elliptique, chacun de ce mâchoires comprenant une griffe inférieure (12,14), à laquelle une griffe supérieure (13,15) est fixée comme un élément à arcade, ayant la même courbure elliptique de la griffe inférieure (12,14) et légèrment déportée vers l'intérieur au regard de ce dernière et en ce que lesdites griffes supérieures (13,15) ont une section transversale circulaire et lesdites griffes inférieures (12,14) ont une surface intérieure ayant un profil légèrment concave vers l'intérieure.

2. Pince pour soutenir un anneau d'anastomose d'intestin selon la revendication 1, caractérisée en ce que, du point de vue géométrique les griffes supérieures et inférieures (12,14; 13,15) ont une telle courbure, que dans leur position fermée, les cercles inscrits dans eux et tangents à leur profils intérieurs, sont concentriques.
